(19) Europäisches Patentamt
European Patent Office
Office européen des brevets

(11) Numéro de publication : **0 407 256 B1**

(12) # FASCICULE DE BREVET EUROPEEN

(45) Date de publication du fascicule du brevet : **19.05.93 Bulletin 93/20**

(51) Int. Cl.$^5$ : **B01J 31/24,** C07D 295/02, C07C 247/10

(21) Numéro de dépôt : **90401739.9**

(22) Date de dépôt : **20.06.90**

(54) **Procédé pour réaliser des substitutions nucléophiles.**

(30) Priorité : **22.06.89 FR 8908318**

(43) Date de publication de la demande : **09.01.91 Bulletin 91/02**

(45) Mention de la délivrance du brevet : **19.05.93 Bulletin 93/20**

(84) Etats contractants désignés : **AT BE CH DE DK ES FR GB GR IT LI LU NL SE**

(56) Documents cités :
**EP-A- 0 315 318**
**FR-A- 2 366 237**
**FR-A- 2 617 163**
**US-A- 4 243 829**
**US-A- 4 362 670**
**US-A- 4 786 623**

(73) Titulaire : **RHONE-POULENC SANTE**
**20, avenue Raymond Aron**
**F-92160 Antony (FR)**

(72) Inventeur : **Sinou, Denis**
**12 rue du Docteur Bonhomme**
**F-69003 Lyon (FR)**

(74) Mandataire : **Le Pennec, Magali et al**
**RHONE-POULENC SANTE Service Brevets Santé, 20 Avenue Raymond Aron**
**F-92160 Antony (FR)**

**EP 0 407 256 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé pour réaliser des substitutions nucléophiles en présence d'un système catalytique à base de palladium constitué d'une solution aqueuse d'un dérivé du palladium et d'un ligand hydrosoluble associé à un nitrile

Il est connu, par exemple, de faire réagir des réactifs nucléophiles sur des dérivés allyliques tels que l'acétate de l'alcool cinnamique ou l'acétate de géraniol en présence de catalyseurs choisis parmi les complexes du palladium éventuellement en présence de ligands tels que la triphénylphosphine [J. Tsuji, Tetrahedron, 42, 4361-4401 (1986)].

Il est également connu d'effectuer des réactions de télomérisation de diènes en phase homogène organique, en présence de complexes du palladium et de ligands non-hydrosolubles (FR 2 617 163).

Il est encore connu d'effectuer des réactions de télomérisation de diènes avec des composés à hydrogène mobile (FR 2 366 237, EP 287066) ou des réactions de carbonylation [J. Kiji et coll, Chem. Letters, 957-960 (1988)] mettant en oeuvre des catalyseurs constitués d'un métal de transition associé à une phosphine soluble. Cependant la mise en oeuvre de ces procédés ne permet pas de séparer facilement le catalyseur, qui est soluble dans les solvants organiques utilisés, des produits de la réaction. Il en résulte, d'une part, que la séparation des produits de réaction est difficile et, d'autre part, que le catalyseur ne peut pas être facilement récupéré et recyclé.

Il a maintenant été trouvé, et c'est ce qui constitue l'objet de la présente invention, un procédé pour réaliser des substitutions nucléophiles dans lequel on fait réagir un nucléophile Nu choisi parmi les composés à groupement méthylène actif, les azides, les cyanures, les phénates, les thiolates, les sulfinates, les amines primaires ou secondaires, les hydroxylamines et les formiates sur un fruit de formule générale:

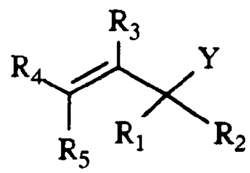

dans laquelle $R_1$, $R_2$, $R_3$, $R_4$ et $R_5$, identiques ou différents, représentent un atome d'hydrogène ou un radical hydrocarboné aliphatique éventuellement fonctionnalisé, ou un radical aromatique ou un radical cyano ou un radical alcanoyloxy, étant entendu:

que $R_1$ et $R_5$ peuvent former ensemble un radical alkylène contenant 2 ou 3 atomes de carbone, ou

que $R_3$ et $R_4$ peuvent former ensemble une liaison, ou

que $-C(R_1R_2)Y$ peuvent former l'enchaînement

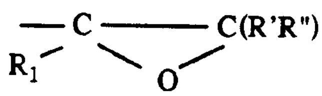

dans lequel R'et R", identiques ou différents, représentent un atome d'hydrogène ou un radical alkyle contenant 1 à 4 atomes de carbone, et Y présente un atome d'halogène ou un radical -OR, -SR, -O-CO-OR, O-CO-N(R)$_2$, -O-PO(OR)$_2$, -NR$_2$, -NO$_2$, -SO$_2$R, -N(R$_3$)X, -S(R)$_2$X ou

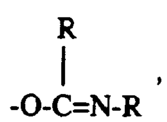

R représentant un radical alkyle contenant 1 à 4 atomes de carbone et X représentant un anion, caractérisé en ce que l'on opère en présence d'un catalyseur constitué d'une solution aqueuse d'un dérivé du palladium et d'un ligand hydrosoluble associé à un nitrile.

Dans ce qui précède et ce qui suit, on entend par radical hydrocarboné aliphatique, un radical alkyle contenant 1 à 20 atomes de carbone ou un radical alkényle contenant 2 à 20 atomes de carbone et éventuellement plusieurs doubles liaisons conjuguées ou non.

Le réactif nucléophile Nu pouvant réagir sur le produit de formule générale (I) est choisi parmi les composés capables de générer un anion.

Plus particulièrement, le nucléophile est choisi parmi les composés à groupement méthylène actif (acétylacétate de méthyle ou d'éthyle), les azides (azidure de sodium), les cyanures (cyanure de sodium), les phénates (phénate de sodium), les thiolates, les sulfinates, les amines primaires ou secondaires, les hydroxylamines ou les formiates.

Le composé de palladium utilisable selon le procédé de l'invention doit être soluble dans l'eau ou être capable de passer en solution dans l'eau par réaction de coordination avec le ligand hydrosoluble.

Les composés du palladium peuvent être choisis parmi les dérivés du palladium à un degré d'oxydation zéro ou différent de zéro.

Les composés du palladium dans lesquels le palladium se trouve à un état d'oxydation différent de zéro sont, par exemple, les sels d'acides organiques (acétate) et les carbonate, borate, bromure, chlorure, iodure, hydroxyde, nitrate, sulfate, citrate, alkyl- ou arylsulfonate, acétylacétonate de palladium, le chlorure de palladium bis-benzonitrile, le tétrachloropalladate de potassium ou les chlorure ou acétate de $\pi$-allyle palladium.

Les composés du palladium dans lesquels le palladium est à l'état d'oxydation zéro sont, par exemple, le tétra-triphénylphosphine palladium zéro, le bis-dibenzylidèneacétone palladium zéro ou le bis-cyclooctadiène-1,5 palladium zéro.

Généralement, on utilise une quantité de palladium ou de composé du palladium telle que le nombre d'atome-grammes de palladium élémentaire par litre de solution catalytique aqueuse soit compris entre $10^{-4}$ et 1 et, de préférence entre $10^{-3}$ et 0,5.

Les ligands hydrosolubles sont choisis parmi les phosphines solubles dans l'eau qui sont décrites dans le brevet français FR 76 22824 (2 366 237) ou dans le brevet français FR 83 12468 (2 549 840).

D'un intérêt tout particulier est la tri-métasulfotriphénylphosphine (TPPTS) sous forme de sel de sodium (TPPTS Na).

Généralement, on utilise une quantité de ligand telle que le nombre d'atome-grammes de phosphore trivalent rapporté à un atome-gramme de palladium soit compris entre 1 et 200 et, de préférence, entre 3 et 100.

Le nitrile qui est associé à la solution aqueuse du composé du palladium est de préférence le benzonitrile ou le butyronitrile.

Généralement, on utilise un volume de nitrile représentant de 0,01 à 100 fois le volume de la solution aqueuse du composé du palladium, et de préférence de 0,1 à 10 fois.

Pour la mise en oeuvre du procédé selon l'invention, il peut être avantageux d'ajouter la solution aqueuse du composé du palladium à la solution des réactifs dans le nitrile.

Le procédé selon l'invention peut être utilisé pour réaliser en milieu biphasique les réactions, mettant en oeuvre un catalyseur à base de palladium, habituellement réalisées en milieu organique homogène.

Lorsque l'on fait réagir un nucléophile Nu sur un produit de formule générale (I) dans laquelle Y est défini comme précédemment et les symboles $R_1$, $R_2$, $R_3$, $R_4$ et $R_5$, identiques ou différents, représentent un atome d'hydrogène ou un radical hydrocarboné aliphatique ou un radical aromatique, les radicaux $R_1$ et $R_5$ pouvant former un radical alkylène contenant 2 ou 3 atomes de carbone, on obtient les produits de formule:

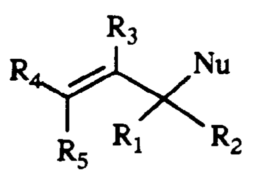

ou

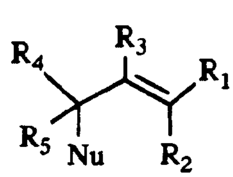

Lorsque l'on fait réagir un nucléophile Nu' choisi parmi "l'hydrogène", un alcool ou une amine primaire ou secondaire sur un produit de formule générale (I) en présence d'oxyde de carbone, on obtient un produit de formule générale:

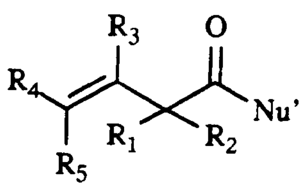

ou

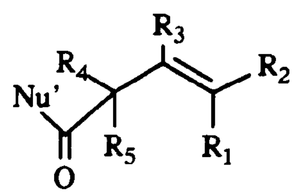

Lorsque l'on fait réagir un nucléophile $Nu_1$ sur un produit de formule générale :

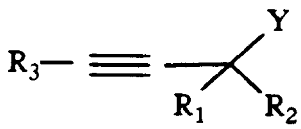

(II)

dans laquelle Y est défini comme précédemment et $R_1$, $R_2$ et $R_3$, identiques ou différents, représentent un atome d'hydrogène ou un radical hydrocarboné aliphatique ou un radical aromatique, on obtient un produit de formule générale:

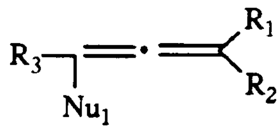

sur lequel on peut faire réagir un excès du même nucléophile ou un nucléophile différent $Nu_2$ pour obtenir un produit de formule générale:

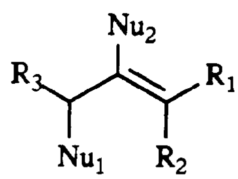

Lorsque l'on fait réagir un nucléophile Nu sur un produit de formule générale :

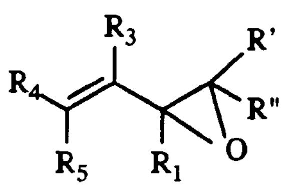

(III)

dans laquelle $R_1$, $R_3$, $R_4$ et $R_5$, identiques ou différents, représentent un atome d'hydrogène ou un radical hydrocarboné aliphatique ou un radical aromatique et R' et R", identiques ou différents, représentent un atome d'hydrogène ou un radical alkyle contenant 1 à 4 atomes de carbone, on obtient un produit de formule générale:

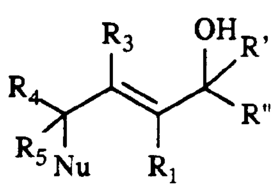

ou

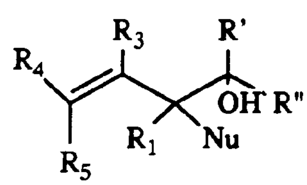

Généralement le procédé est mis en oeuvre en ajoutant la solution aqueuse catalytique à la solution du produit de formule générale (I), (II) ou (III) et du nucléophile Nu dans le nitrile puis en chauffant le mélange agité à une température inférieure à 200°C.

La solution aqueuse catalytique peut être séparée in situ en mélangeant dans l'eau le dérivé du palladium et le ligand en opérant sous atmosphère inérte (azote, argon).

Le mélange réactionnel peut être chauffé avant ou après l'introduction du produit de formule générale (I), (II) ou (III) qui peut lui-même être introduit avant ou après le nucléophile Nu ou simultanément.

Le rapport moléculaire du nucléophile Nu au produit de formule générale (I), (II) ou (III) n'est pas critique. Cependant il est particulièrement avantageux d'opérer en présence d'un léger excès molaire du nucléophile Nu.

Généralement le procédé est mis en oeuvre à une température inférieure à 200°C et de préférence entre 20 et 100°C.

A la fin de la réaction, et après refroidissement à une température voisine de 20°C, le mélange réactionnel constitué de deux phases non miscibles est séparé par décantation en une phase aqueuse contenant le système catalytique et en une phase organique contenant le produit de la réaction.

La phase aqueuse peut être utilisée pour catalyser une nouvelle action d'un produit de formule générale (I), (II) ou (III) sur le nucléophile Nu.

Les produits obtenus selon le procédé de la présente invention sont particulièrement utiles comme intermédiares en synthèse organique pour la préparation de parfums ou de vitamines. Par exemple, le produit de condensation du carbonate d'éthyle et de géranyle sur l'acétylacétate de méthyle permet de préparer la géranylacétone selon Tsuji et coll., J. Org. Chem., 50, 1523 (1985). Les produits obtenus selon le procédé de la présente invention peuvent aussi être utilisés pour préparer la vitamine A selon le procédé décrit dans le brevet français FR 2 589 862. D'autres applications sont décrites, par exemple, dans l'article de J. Tsuji, J. Organometal. Chem., 300, 281-305 (1986).

Les exemples suivants, donnés à titre non limitatif, montrent comment l'invention peut être mise en pratique.

## EXEMPLE 1

Sous atmosphère d'argon, on dissout 33,6 mg d'acétate de palladium [$Pd(OAc)_2$] soit 0,15 m.At.g de palladium et 735 mg de TPPTS Na soit 1,2 m.At.g de $P^{3+}$ dans 5 cm3 d'eau dégazée. Cette solution est injectée dans un réacteur contenant 618 mg de carbonate d'éthyle et de cinnamyle (3 mmoles) et 585 mg d'acétylacétate d'éthyle (4,5 mmoles) dans 5 cm3 de benzonitrile. On agite pendant 18 heures à 50°C. Après refroidissement, le mélange réactionnel est constitué d'une phase aqueuse orangé clair et d'une phase organique jaune clair.

La phase organique est séparée et concentrée sous pression réduite. On obtient ainsi 450 mg d'une huile jaune foncé qui est purifiée par chromatographie sur colonne de silice 60 en éluant avec un mélange hexane-acétate d'éthyle.

On obtient ainsi 440 mg de phényl-5 acétyl-2 pentène-4 oate d'éthyle dont la structure est confirmée par le spectre infra-rouge, le spectre de résonance magnétique nucléaire du proton et du $^{13}C$ et l'analyse élémentaire.

Le taux de transformation du carbonate d'éthyle et de cinnamyle est de 94 % et la sélectivité en produit de monosubstitution est voisine de 100 %.

Dans un réacteur contenant la phase aqueuse obtenue précédemment, on ajoute 618 mg de carbonate d'éthyle et de cinnamyle (3 mmoles) et 585 mg d'acétylacétate d'éthyle (4,5 mmoles) dans 5 cm3 de benzonitrile. On agite pendant 18 heures à 50°C. En traitant le mélange réactionnel de la même manière que précédemment, on obtient 413 mg de phényl-5 acétyl-2 pentène-4 oate d'éthyle.

Le taux de transformation du carbonate d'éthyle et de cinnamyle est de 93 % et la sélectivité en produit de monosubstitution est de 100 %.

En effectuant un second recyclage dans les conditions décrites précédemment, on obtient le produit de mono-substitution avec une sélectivité de 100 %, le taux de transformation du carbonate d'éthyle et de cinnamyle étant voisin de 100 %.

## EXEMPLES COMPARATIFS

Dans le tableau I sont rassemblés les résultats obtenus en mettant en oeuvre le système catalytique dans les conditions décrites ci-dessus en l'absence d'un des constituants.

TABLEAU I

| Catalyseur | Ligand | Solvant | Taux de transformation (%) |
|---|---|---|---|
| $Pd(dba)_2$ | TPPTS | oxyde d'isopropyle | 80 * |
| $Pd(dba)_2$ | TPPTS | oxyde de n.butyle | 0 * |
| - | - | benzonitrile | 0 |
| $Pd(dba)_2$ | - | benzonitrile | 0 |
| - | TPPTS | benzonitrile | 0 |

* apparition d'un dépôt métallique de palladium

EXEMPLE 2

Sous atmosphère d'argon, on dissout 28 mg de bis-dibenzylidèneacétone palladium zéro [$Pd(dba)_2$] soit 0,05 m.At.g de palladium et 172 mg de TPPTS Na soit 0,28 m.At.g de $P^{3+}$ dans 3 cm3 d'eau dégazée. Cette solution est injectée dans un réacteur contenant 211 mg de carbonate d'éthyle et de cinnamyle (1 mmole) et 200 mg d'acétylacétate d'éthyle (1,54 mmole) dans 3 cm3 de benzonitrile. On agite pendant 18 heures à 50°C. Le mélange réactionnel est traité dans les conditions de l'exemple 1.

On obtient ainsi 176 mg de phényl-5 acétyl-2 pentène-4E oate d'éthyle.

Le taux de transformation du carbonate d'éthyle et de cinnamyle est de 100 %.

La sélectivité en produit de mono-substitution est de 100%.

A la phase aqueuse obtenue précédemment, on ajoute 208 mg de carbonate d'éthyle et de cinnamyle (1 mmole) et 204 mg d'acétylacétate d'éthyle (1,57 mmole). On agite pendant 18 heures à 50°C.

On traite le mélange réactionnel dans les conditions décrites dans l'exemple 1.

On obtient ainsi 130 mg de phényl-5 acétyl-2 pentène-4E oate d'éthyle.

Le taux de transformation du carbonate d'éthyle et de cinnamyle est de 60 %.

La sélectivité en produit de mono-substitution est de 100%.

Dans le tableau II, sont rassemblés les résultats obtenus au cours des recyclages suivants :

TABLEAU II

| Recyclage | Taux de transformation (%) * | Rendement (%) par rapport au carbonate consommé ** |
|---|---|---|
| N° 2 | 75 | 62 |
| N° 3 | 64 | 58 |
| N° 4 | 54 | 45 |

* détermination à partir du spectre de résonance magnétique nucléaire

** détermination après séparation sur colonne

EXEMPLE 3

Sous atmosphère d'argon, on dissout 27 mg de $[Pd(OAc)_2]$ soit 0,12 m.At.g de palladium, 290 mg de TPPTS Na soit 0,50 m.At.g de $P^{3+}$ et 695 mg d'azidure de sodium. Cette solution est injectée dans un réacteur contenant 455 mg d'acétate de cinnamyle (2,6 mmoles) dans 3 cm3 de benzonitrile. On agite pendant 3 heures à 50°C. Après refroidissement, le mélange réactionnel est traité dans les conditions de l'exemple 1.

La phase organique séparée et concentrée sous pression réduite fournit 330 mg d'azido-1 phényl-3 propène-2 dont la structure est confirmée par le spectre infra-rouge, le spectre de résonance magnétique nucléaire du proton et l'analyse élémentaire.

Le taux de transformation de l'acétate de cinnamyle est de 100 % et la sélectivité en azide est de 100 %.

Dans le réacteur contenant la phase aqueuse, on ajoute 445 mg d'acétate de cinnamyle (2,5 mmoles) dans 3 cm3 de benzonitrile. On agite pendant 3 heures à 50°C.

Le mélange réactionnel est traité comme précédent. On obtient ainsi 400 mg d'azido-1 phényl-3 propène-2.

Le taux de transformation de l'acétate de cinnamyle est voisin de 100 % et la sélectivité en azide est voisine de 100 %.

EXEMPLE 4

Sous atmosphère d'argon, on dissout 24 mg de $[Pd(OAc)_2]$ soit 0,1 m.At.g de palladium et 422 mg de TPPTS Na soit 0,7 m.At.g de $P^{3+}$ dans 5 cm3 d'eau dégazée. Cette solution est injectée dans un réacteur contenant 467 mg de carbonate d'éthyle et de géranyle et 556 mg d'acétylacétate d'éthyle (3,6 mmoles) dans 5 cm3 de benzonitrile. On agite pendant 24 heures à 50°C. Après refroidissement, le mélange réactionnel est traité dans les conditions de l'exemple 1.

La phase organique séparée et concentrée sous pression réduite fournit 440 mg d'une huile jaune foncée dont l'analyse par résonance magnétique nucléaire du proton et par chromatographie en phase vapeur montre qu'elle est constituée de :

55 % de

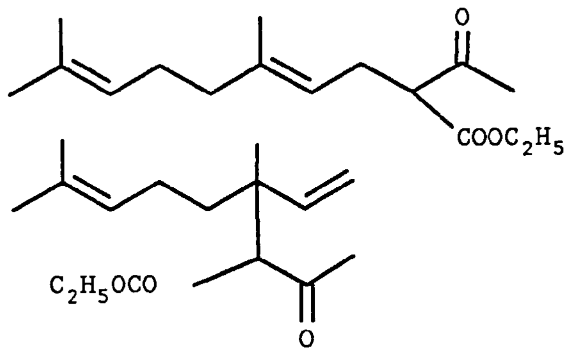

(E)

37 % de

8 % de

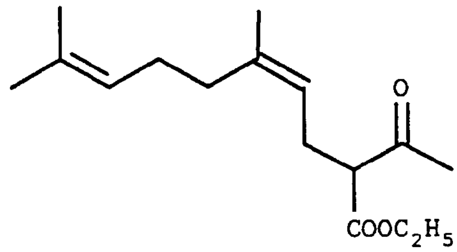

(Z)

Le taux de transformation du carbonate d'éthyle et de géranyle est de 100 %.

EXEMPLE 5

Sous atmosphère d'argon, on dissout 31 mg de $[Pd(OAc)_2]$ soit 0,14 m.At.g de palladium et 582 mg de TPPTS Na soit 0,95 m.At.g de $P^{3+}$ dans 5 cm3 d'eau dégazée. Cette solution est injectée dans un réacteur contenant 427 mg de carbonate d'éthyle et de cinnamyle (2,0 mmoles) et 274 mg de morpholine (2,7 mmoles) dans 5 cm3 de benzonitrile. On agite pendant 18 heures à 50°C. Le mélange réactionnel est traité dans les conditions de l'exemple 1.

La phase organique séparée et concentrée sous pression réduite fournit 350 mg d'une huile dont l'analyse par résonance magnétique nucléaire montre qu'elle est constituée de (phényl-3 propène-2 yl-1)-1 morpholine.

Le taux de transformation du carbonate d'éthyle et de cinnamyle est de 100 % et la sélectivité en produit de mono-substitution est de 100 %.

## Revendications

1. Procédé pour réaliser des substitutions nucléophiles dans lequel on fait réagir un nucléophile Nu choisi parmi les composés à groupement méthylène actif, les azides, les cyanures, les phénates, les thiolates, les sulfinates, les amines primaires ou secondaires, les hydroxylamines et les formiates sur un produit de formule générale :

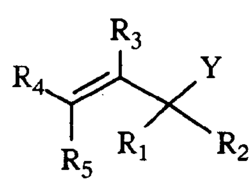

dans laquelle $R_1$, $R_2$, $R_3$, $R_4$ et $R_5$, identiques ou différents, représentent un atome d'hydrogène ou un radical hydrocarboné aliphatique éventuellement fonctionnalisé, ou un radical aromatique ou un radical cyano ou un radical alcanoyloxy, étant entendu :
que $R_1$ et $R_5$ peuvent former ensemble un radical alkylène contenant 2 ou 3 atomes de carbone, ou
que $R_3$ et $R_4$ peuvent former ensemble une liaison, ou
que $-C(R_1R_2)Y$ peuvent former l'enchaînement

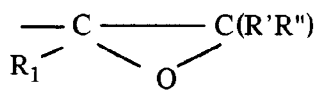

dans lequel R'et R", identiques ou différents, représentent un atome d'hydrogène ou un radical alkyle contenant 1 à 4 atomes de carbone, et
Y représente un atome d'halogène ou un radical -OR, -SR, -O-CO-OR, O-CO-N(R)$_2$, -O-PO(OR)$_2$, -NR$_2$, -NO$_2$, -SO$_2$R, -N(R$_3$)X, -S(R)$_2$X ou

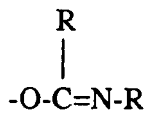 ,

R représentant un radical alkyle contenant 1 à 4 atomes de carbone et X représentant un anion, caractérisé en ce que l'on opère en présence d'un catalyseur constitué d'une solution aqueuse d'un dérivé du palladium et d'un ligand hydrosoluble associé à un nitrile.

2. Procédé selon la revendication 1 caractérisé en ce que le dérivé du palladium est choisi parmi les dérivés du palladium zéro ou d'un degré d'oxydation différent de zéro.

3. Procédé selon la revendication 2 caractérisé en ce que le dérivé du palladium est choisi parmi les sels d'acides organiques et les carbonate, borate, bromure, chlorure, iodure, hydroxyde, nitrate, sulfate, citrate, alkyl- ou arylsulfonate, acétylacétonate de palladium, le chlorure de palladium bisbenzonitrile, le tétrachloropalladate de potassium, les chlorure ou acétate de $\pi$-allyl palladium, la tétra-triphénylphosphine palladium zéro, le bis-dibenzylidèneacétone palladium zéro et le bis-cyclooctadiène-1,5 palladium zéro.

4. Procédé selon la revendication 1 caractérisé en ce que le ligand hydrosoluble est choisi parmi les phosphines ou diphosphines sulfonées.

5. Procédé selon la revendication 4 caractérisé en ce que le ligand est la tri-métasulfotriphénylphosphine éventuellement sous forme de sel de sodium.

6. Procédé selon la revendication 1 caractérisé en ce que le nitrile est choisi parmi le benzonitrile et le butyronitrile.

7. Procédé selon la revendication 1 caractérisé en ce que la solution catalytique aqueuse contient de $10^{-4}$ à 1 atome-grammes de palladium par litre.

8. Procédé selon la revendication 1 caractérisé en ce que la solution catalytique aqueuse contient entre 1 et 200 moles de ligand par atome-gramme de palladium.

9. Procédé selon l'une des revendications 4 ou 5 caractérisé en ce que la solution catalytique aqueuse contient de 1 à 200 atome-grammes de phosphore trivalent par atome-gramme de palladium.

10. Procédé selon la revendication 1 caractérisé en ce que le nitrile représente, en volumes, une quantité représentant 0,01 à 100 fois celui de la solution catalytique aqueuse.

11. Procédé selon l'une quelconque des revendications précédentes caractérisé en ce que la température de réaction est inférieure à 200°C.

12. Procédé selon la revendication 11 caractérisé en ce que la température de réaction est comprise entre 20 et 100°C.

13. Procédé selon l'une quelconque des revendications précédentes caractérisé en ce que en fin de réaction, on sépare le produit de la réaction par décantation et on récupère la solution aqueuse catalytique qui peut être recyclée.

## Claims

1. Process for carrying out nucleophilic substitutions in which process a nucleophile Nu, chosen from compounds containing an active methylene group, azides, cyanides, phenoxides, thiolates, sulphinates, primary or secondary amines, hydroxylamines and formates, is reacted with a product of general formula:

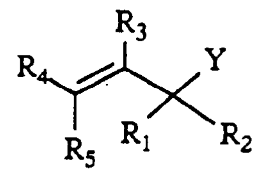

in which $R_1$, $R_2$, $R_3$, $R_4$ and $R_5$, which are identical or different, represent a hydrogen atom or an optionally functionalised, aliphatic hydrocarbon radical or an aromatic radical or a cyano radical or an alkanoyloxy radical, it being understood:
that $R_1$ and $R_5$ can together form an alkylene radical containing 2 or 3 carbon atoms, or
that $R_3$ and $R_4$ can together form a bond, or
that $-C(R_1R_2)$ Y can form the linkage

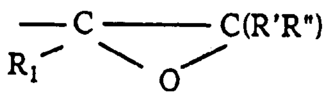

in which R' and R", which are identical or different, represent a hydrogen atom or an alkyl radical containing 1 to 4 carbon atoms, and
Y represents a halogen atom or a radical -OR, -SR, -O-CO-OR, $O-CO-N(R)_2$, $-O-PO(OR)_2$, $-NR_2$, $-NO_2$, $-SO_2R$, $-N(R_3)X$, $-S(R)_2X$ or

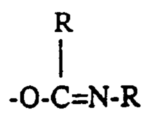 ,

R representing an alkyl radical containing 1 to 4 carbon atoms and X representing an anion, characterised in that the reaction is carried out in the presence of a catalyst consisting of an aqueous solution of a palladium derivative and a water-soluble ligand in combination with a nitrile.

2. Process according to Claim 1, characterised in that the palladium derivative is chosen from derivatives of palladium (0) or the derivatives with an oxidation number other than zero.

3. Process according to Claim 2, characterised in that the palladium derivative is chosen from palladium carbonate, borate, bromide, chloride, iodide, hydroxide, nitrate, sulphate, citrate, alkyl- or arylsulphonate, acetylacetonate and organic acid salts, bis(benzonitrile)palladium chloride, potassium tetrachloropalladate, ($\pi$-allyl)palladium chloride or acetate, tetra(triphenylphosphine)palladium(0), bis(dibenzylideneacetone)palladium(0) and bis(1,5-cyclooctadiene)palladium(0).

4. Process according to Claim 1, characterised in that the water-soluble ligand is chosen from sulphonated phosphines or diphosphines.

5. Process according to Claim 4, characterised in that the ligand is tri(metasulpho)triphenylphosphine, optionally in the sodium salt form.

6. Process according to Claim 1, characterised in that the nitrile is chosen from benzonitrile and butyronitrile.

7. Process according to Claim 1, characterised in that the aqueous catalytic solution contains from $10^{-4}$ to 1 gram-atoms of palladium per litre.

8. Process according to Claim 1, characterised in that the aqueous catalytic solution contains between 1

and 200 mol of ligand per gram-atom of palladium.

**9.** Process according to one of Claims 4 or 5, characterised in that the aqueous catalytic solution contains from 1 to 200 gram-atoms of trivalent phosphorus per gram-atom of palladium.

**10.** Process according to Claim 1, characterised in that the nitrile represents, by volume, a quantity representing 0.01 to 100 times that of the aqueous catalytic solution.

**11.** Process according to any one of the preceding claims, characterised in that the reaction temperature is below 200°C.

**12.** Process according to Claim 11, characterised in that the reaction temperature is between 20 and 100°C.

**13.** Process according to any one of the preceding claims, characterised in that, at the end of the reaction, the product of the reaction is separated by settling and the aqueous catalytic solution is recovered and can be recycled.

**Patentansprüche**

**1.** Verfahren zur Durchführung von nucleophilen Substitutionen, bei dem man ein Nucleophiles Nu, ausgewählt unter den Verbindungen mit aktiver Methylengruppe, den Aziden, den Cyaniden, den Phenaten, den Thiolaten, den Sulfinaten, den primären oder sekundären Aminen, den Hydroxylaminen und den Formiaten, mit einem Produkt der allgemeinen Formel

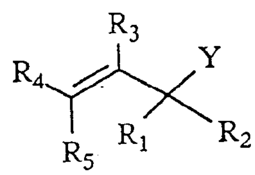

umsetzt, worin $R_1$, $R_2$, $R_3$, $R_4$ und $R_5$, die identisch oder voneinander verschieden sind, für ein Wasserstoffatom oder einen gegebenenfalls funktionalisierten aliphatischen Kohlenwasserstoffrest oder einen aromatischen Rest oder eine Cyanogruppe oder einen Alkanoyloxyrest stehen, mit der Maßgabe, daß:
$R_1$ und $R_5$ gemeinsam einen Alkylenrest mit 2 oder 3 Kohlenstoffatomen bilden können, oder daß
$R_3$ und $R_4$ gemeinsam eine Bindung bilden können, oder daß -C($R_1R_2$)Y die Verknüpfung

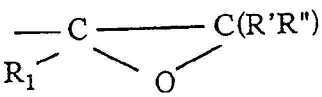

bilden können, worin R' und R", die identisch oder voneinander verschieden sind, für ein Wasserstoffatom oder einen Alkylrest mit 1 bis 4 Kohlenstoffatomen stehen, und Y ein Halogenatom oder einen Rest -OR, -SR, -O-CO-OR, O-CO-N(R)$_2$, -O-PO(OR)$_2$, -NR$_2$, -NO$_2$, SO$_2$R, -N(R$_3$)X, -S(R)$_2$X oder

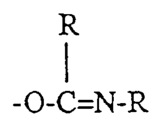

wiedergibt, wobei R einen Alkylrest mit 1 bis 4 Kohlenstoffatomen bedeutet, und X ein Anion darstellt, dadurch gekennzeichnet, daß man in Anwesenheit eines Katalysators arbeitet, der aus einer wäßrigen Lösung eines Palladiumderivats und eines mit einem Nitril assoziierten wasserlöslichen Liganden besteht.

**2.** Verfahren gemäß Anspruch 1, dadurch gekennzeichnet, daß das Palladiumderivat unter den Derivaten des Palladium-(Null) oder mit einem von Null verschiedenen Oxidationsgrad ausgewählt wird.

**3.** Verfahren gemäß Anspruch 2, dadurch gekennzeichnet, daß das Palladiumderivat unter den Salzen der

organischen Säuren und dem Carbonat, Borat, Bromid, Chlorid, Jodid, Hydroxid, Nitrat, Sulfat, Citrat, Alkyl- oder Arylsulfonat, Acetylacetonat des Palladiums, dem Palladium-bis-benzonitrilchlorid, dem Kaliumtetrachloropalladat, dem $\pi$-Allylpalladium-chlorid oder -acetat, dem Tetra-triphenylphosphinpalladium-(Null), dem Bis-dibenzylidenaceton-palladium-(Null) und dem Bis-1,5-cyclooctadien-palladium-(Null) ausgewählt wird.

**4.** Verfahren gemäß Anspruch 1, dadurch gekennzeichnet, daß der wasserlösliche Ligand unter den sulfonierten Phosphinen oder Diphosphinen ausgewählt wird.

**5.** Verfahren gemäß Anspruch 4, dadurch gekennzeichnet, daß der Ligand das Tri-metasulfotriphenylphosphin, gegebenenfalls in Form des Natriumsalzes, ist.

**6.** Verfahren gemäß Anspruch 1, dadurch gekennzeichnet, daß das Nitril unter Benzonitril und Butyronitril ausgewählt wird.

**7.** Verfahren gemäß Anspruch 1, dadurch gekennzeichnet, daß die wäßrige katalytische Lösung $10^{-4}$ bis 1 Grammatom Palladium je Liter enthält.

**8.** Verfahren gemäß Anspruch 1, dadurch gekennzeichnet, daß die wäßrige katalytische Lösung zwischen 1 und 200 Mol Liganden je Grammatom Palladium enthält.

**9.** Verfahren gemäß einem der Ansprüche 4 oder 5, dadurch gekennzeichnet, daß die wäßrige katalytische Lösung 1 bis 200 Grammatom 3-wertigen Phosphor je Grammatom Palladium enthält.

**10.** Verfahren gemäß Anspruch 1, dadurch gekennzeichnet, daß das Nitril in Volumina eine Menge entsprechend dem 0,01-bis 100fachen derjenigen der katalytischen wäßrigen Lösung ausmacht.

**11.** Verfahren gemäß einem der vorhergehenden Ansprüche, dadurch gekennzeichnet, daß die Reaktionstemperatur niedriger als 200°C ist.

**12.** Verfahren gemäß Anspruch 11, dadurch gekennzeichnet, daß die Reaktionstemperatur zwischen 20 und 100°C liegt.

**13.** Verfahren gemäß einem der vorhergehenden Ansprüche, dadurch gekennzeichnet, daß man am Ende der Reaktion das Reaktionsprodukt durch Dekantieren abtrennt und die katalytische wäßrige Lösung, die recyclisiert werden kann, zurückgewinnt.